# EUROPEAN PATENT SPECIFICATION

(11) **EP 2 168 535 B1**
(45) Date of publication and mention of the grant of the patent: **16.07.2014**
(21) Application number: 09171784.3
(22) Date of filing: 30.09.2009
(51) Int. Cl.: A61F 2/16

(54) **Intraocular lens injection instrument**
Injektor für Intraokularlinsen
Injecteur pour lentilles intraoculaires

(30) Priority: 30.09.2008 JP 2008255827
(43) Date of publication of application: 31.03.2010
(73) Proprietor: NIDEK CO., LTD, Gamagori-shi Aichi 443-0038 (JP)
(72) Inventor: Nagasaka, Shinji, Gamagori-shi Aichi 443-0038 (JP)
(74) Representative: Prüfer & Partner GbR European Patent Attorneys

(56) References cited:
- EP-A2- 1 360 944
- WO-A1-97/15253
- US-A1- 2001 001 822
- US-A1- 2005 149 057

## Description

### Technical Field

The present invention relates to an intraocular lens injection instrument for injecting an intraocular lens in an eye.

### Background Art

As one of operative treatments for cataract, heretofore, there has generally been used a method in which a lens is removed from a patient's eye and then a foldable, soft intraocular lens (IOL) is injected in place of the lens. For injection of such foldable IOL, an IOL injection instrument called an injector is used. With this injector, the IOL is injected in a folded state into the eye through an incision. The incision therefore has only to be formed in advance with a minimum diameter in the eye. This injector is configured to advance the IOL set therein by a push rod called a plunger while the IOL is folded to a smaller size, and push out the IOL through a tip end of the injector. When the IOL is to be injected into an eye by use of such injector, there is a case where the posture of the plunger could not be controlled and becomes deviated, and thus the IOL could not be appropriately pushed out. To avoid such disadvantage, an IOL injection instrument provided with a member for preventing axis deflection of the plunger has been proposed (Patent Literature 1). The IOL injection instrument disclosed in Patent Literature 1 is configured such that a posture control member for preventing axis deflection and movable in interlocking relation with a plunger is placed near the front end of the plunger. The interlocking is released during a moving operation of a lens so that a supporting point of centering is placed as far front as possible, thereby restraining axis deflection. In regard to this IOL injection instrument, before the lens is fed out of the front end, it is necessary to make a final check whether a push-out axis of the plunger pushing the IOL lens does not deflect and the lens is appropriately moved forward without displacement. This checking is conducted by temporarily stopping the pushing of the IOL lens in a standby position midway in a feeding passage and then checking the states of the IOL lens and the plunger.

US 2001/0001822 A1 describes a deformable intraocular lens insertion system comprising a lens injection device and a lens cartridge with a rotary connecting arrangement between the lens injection device and the lens cartridge. The lens injecting device comprises a cylindrical barrel with a lens cartridge receiver. The lens cartridge assembly is provided such that it can not rotate after being inserted into the lens injecting device.

### Citation List

### Patent Literature

Patent Literature 1: JP2004-24854A

### Summary of Invention

### Technical Problem

However, even the IOL injection instrument disclosed in Patent Literature 1 could not be used in case the IOL lens placed in the standby position is displaced and axis deflection of the plunger is caused during a push-out operation of the IOL lens.

The present invention has been made to solve the above problems and has a purpose to provide an IOL injection instrument capable of making centering of a push rod (a plunger) by a simple configuration, preventing axis deflection, and appropriately pushing out an IOL lens.

This purpose is achieved with an instrument according to claim 1. Advantageous developments are subject to the dependent claims.

### Solution to Problem

To achieve the above purpose, the present invention provides an IOL injection instrument comprising: a lens holding part including: a setting part having a lens set position on which an intraocular lens is placed and a lens standby position provided forward of the lens set position; and a cylindrical injection part coupled to the setting part to feed the lens placed on the setting part from a tip end; a main unit having a cylindrical configuration provided with the lens holding part at a front end; and a plunger provided to be movable forward and backward in an axis direction in a cylinder of the main unit to push the lens out of the lens holding part, **characterized in that** the main unit includes a centering portion internally fixedly formed therein to prevent axis deflection of the plunger, the centering portion having a fitting part having almost the same diameter as that of the plunger, and the centering portion being fittable to the plunger during a moving operation of the plunger to center the plunger, the plunger is formed of such a material and with such a diameter as to produce a restoring force capable of correcting displacement and a bent state of the lens against the friction force caused by contact between the lens placed in the lens standby position and the inner wall of the lens holding part in order to prevent the plunger from bending in a position forward of the centering portion and deflecting an axis by displacement of the lens when the lens is moved from the lens set position to the lens standby position during the moving operation.
Further developments of the present invention are given in the dependent claims.

### Advantageous Effects of Invention

According to the present invention, the centering of the push rod (plunger) can be achieved by a simple configuration, thereby preventing axis deflection and appropriately pushing out the IOL.

### Brief Description of Drawings

FIGs. 1A and 1B are external views an IOL injection instrument in an embodiment;
FIG. 2 is a view showing a configuration of an IOL;
FIGs. 3A to 3C are external views of a cartridge in the embodiment;
FIGs. 4A and 4B are views showing a folding method of the IOL due to deformation of the cartridge 10;
FIG. 5 is an external view of a cylindrical unit;
FIG. 6 is a cross sectional side view of the IOL injection instrument;
FIGs. 7A and 7B are schematic cross sectional views near a centering portion while a head portion is in contact with an optic part;
FIGs. 8A and 8B are schematic cross sectional views near the centering portion while the IOL is being pushed by the head portion; and
FIGs. 9A and 9B are enlarged top views of the cartridge while the IOL is placed in a standby position.

### Description of Embodiments

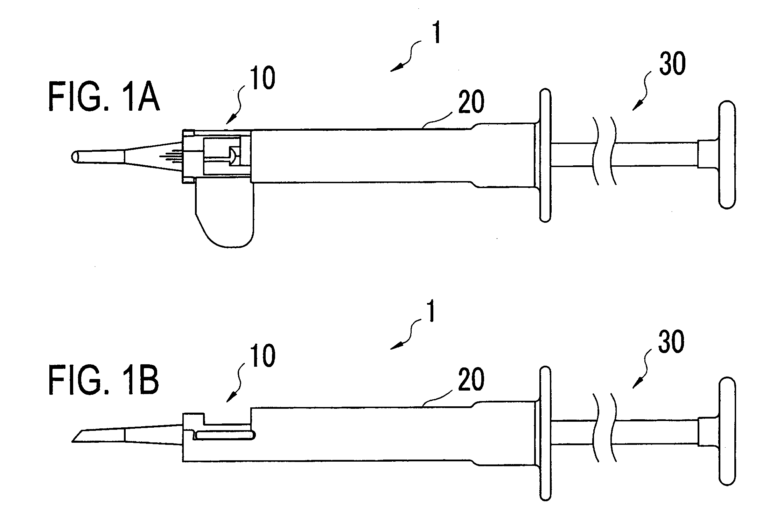

A detailed description of a preferred embodiment of the present invention will now be given referring to the accompanying drawings. FIGs. 1 are a schematic external view of an IOL injection instrument 1 used in this embodiment. Specifically, FIG. 1A is a top view of the IOL injection instrument 1 and FIG. 1B is a side view of the same.

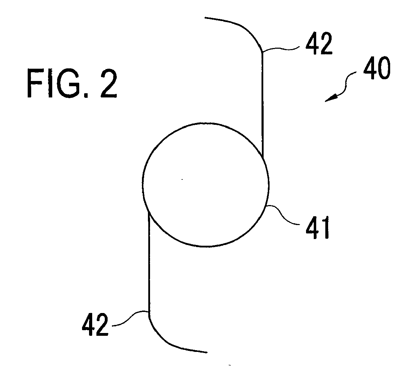

The IOL injection instrument 1 includes, in the order of insertion into an eye, a lens holding part (hereinafter, a "cartridge") 10 for holding an intraocular lens (IOL) 40 (see Fig. 2), the cartridge 10 including an injection part for injecting the IOL 40 into the eye through an incision made in the eye and a setting part in which the IOL 40 is to be set; a cylindrical unit (a main unit) 20 having a front end in which the cartridge 10 is mountable; and a plunger (a push-out member) 30 placed to be movable through the insides of the cartridge 10 and the cylindrical unit 20 and configured to push out the IOL 40 from a tip end of the cartridge 10 mounted in the cylindrical unit 20.

FIG. 2 is a view showing a configuration of the IOL 40. This IOL 40 includes an optic part 41 having predetermined refractive power and a pair of support parts 42, called an open loop, for fixedly supporting the optic part 41 in the eye. The optic part 41 of the IOL 40 used in this embodiment is made of a material commonly used for a typical foldable soft intraocular lens, for example, a monomeric material such as hydroxyethyl methacrylate (HEMA) and a composite material of acrylic ester and methacrylate ester. The support parts 42 are also made of a material commonly used for a typical IOL support part such as polymethylmetacrylate (PMMA). The IOL 40 used in this embodiment is a three-piece IOL produced in such a way that the optic part 41 and the thin-loop-shaped support parts 42 are separately made of the aforementioned materials and then assembled together. The IOL used herein may be configured as a one-piece IOL including an optic part and support parts that are integrally formed in advance.

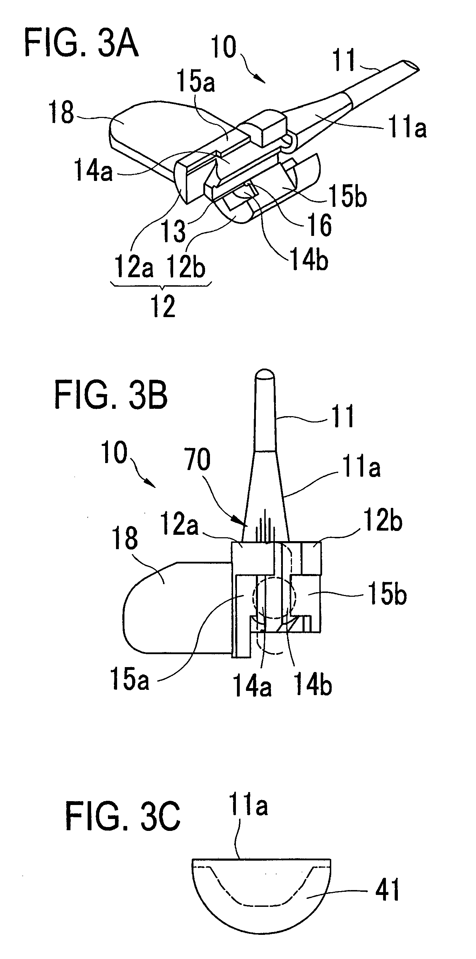

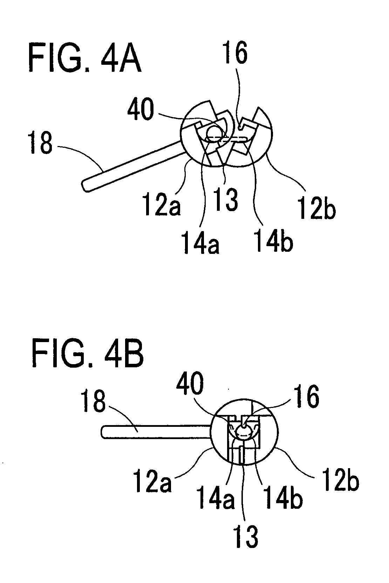

Figs. 3A, 3B, 4A, and 4B are views showing a configuration of the cartridge 10. As shown in the figures, the cartridge 10 integrally includes an injection part 11 having a tapered shape whose diameter becomes smaller (narrower) toward the tip end and a setting part 12 in which the IOL 40 is to be set. It is to be noted that the cartridge 10 is entirely made of synthetic resin as a disposable type which is to be thrown away after one use. Accordingly, the cartridge 10 is preferably made of resin or the like by molding. Furthermore, the cartridge 10 is formed of an optically transparent material to allow a user to externally visually check the state of the IOL 40 placed in the setting part 12 or an injection part 11. In this embodiment, the cartridge 10 is subjected to a polishing process after molding to increase transparency so that the cartridge 10 is almost transparent. Thus, the states of the IOL 40 and the plunger 30 located inside the cartridge 10 can be viewed from outside.

The inside of the injection part 11 is hollow. The folded IOL 40 is fed out through this hollow portion. An inner cylinder 11a located at a base end of the injection part 11 has an inner diameter to make a folded state of the IOL 40 smaller than that in the setting part 12. The inner cylinder 11a has an internal shape so that the IOL 40 is folded in a gentler roll form than at the tip end of the injection part 11. The inner cylinder 11a is a member (a portion) corresponding to a standby position (a lens standby position) during a push-out operation of the IOL 40. The standby position represents the position in which the user checks (visually recognizes) the state of the push-out operation of the IOL injection instrument 1 and the state of the IOL 40. The inner cylinder 11a is formed to have a flat top surface and a side surface curved to the bottom as shown in FIG. 3C so that the cross section perpendicular to the plunger has a D-letter shape. The IOL 40 located in the inner cylinder 11a (in the figure, only the optic part 41 is indicated by a dotted line) is held in a folded state.

The setting part 12 is constituted of two half-split elements 12a and 12b. As shown in Fig. 3A, the half-split elements 12a and 12b are connected to each other at respective lower edges by a hinge 13 so as to be opened and closed. The half-split elements 12a and 12b include setting stages 14a and 14b respectively for mounting thereon the IOL 40. The shapes (wall shapes) of the setting stages 14a and 14b on which the IOL 40 is to be placed are curved in a direction to fold the IOL 40.

When the half-split elements 12a and 12b are closed into contact with each other, the shapes of walls (setting surfaces) of the setting stages 14a and 14b are transformed to substantially conform in cross section to the shape (semi-circle) of an opening of the injection part 11 on a rear end side (see Figs. 4A and 4B). The outer shape of the setting part 12 with the half-split elements 12a and 12b being closed is substantially equal to the shape of an inner wall of the cylindrical unit 20 mentioned later. A region provided by the setting stages 14a and 14b is a place for placing therein the IOL 40 and a set position of an IOL (a lens set position) in this embodiment.

In this embodiment, frictional force between the IOL 40 placed in the set position and the inner wall of the setting part 12 is substantially equal to frictional force between the IOL 40 placed in the standby position and the inner wall of the inner cylinder 11a. This is because during a push-out operation of the IOL 40 a viscoelastic material (or a perfusion) serving as a lubricant is poured into the setting part 12 and the inner cylinder 11a. When the IOL 40 is moved to the standby position, the viscoelastic material applied around the IOL 40 in the set position also enters between the IOL 40 and the inner wall of the inner cylinder 11a. Accordingly, even if the diameter of the inner cylinder 11 is slightly smaller than the inner wall of the setting part 12, the frictional force exerted on the IOL 40 in the standby position is somewhat decreased and thus the frictional force in the set position and the frictional force in the standby position become almost equal. During the push-out operation of the IOL 40, the user can have almost the same feeling in pushing the IOL 40 from the setting position and in pushing the IOL 40 again from the standby position. Thus, the push-out operation can be performed smoothly.

The inner cylinder 11a is formed with a mark 70 serving as a reference for checking the position of the head portion 50 at the front end of a push rod (plunger) 31 mentioned later. The mark 70 includes a straight reference line corresponding to a push-out axis when the mark 70 is viewed from front and several auxiliary lines formed on the right and left of the reference line. Based on those auxiliary lines, the user can easily judge how far the head portion 50 is apart from the reference line. The mark 70 is provided in the form of a groove or protrusion in the outer surface of the injection part 11. The number of lines of the mark 70 is not limited to plural if only the line(s) can act as a reference to judge the position of the head portion 50. The reference line may also be single. The reference line of the mark 70 has only to be so long as to enable checking of the position of the head portion 50. The mark may be any pattern that can be used as a fiducial mark for checking the position of the head portion 50.

Covers 15a and 15b are provided as upper portions of the half-split elements 12a and 12b respectively to cover over the setting stages 14a and 14b when the half-split elements 12a and 12b are closed. The cover 15b is provided at an end with a protrusion 16 protruding from above toward the setting stages 14a and 14b (the hinge 13). The protrusion 16 serves to guide a bending (folding) direction of the IOL 40 set in the cartridge 10 to a direction along the inner walls (the setting surfaces) of the setting stages 14a and 14b. A flat-plate-like grip 18 is provided on the side of the half-split element 12a. The grip 18 will be grasped by a user to hold the cartridge 10.

The cartridge 10 having the above configuration is operated such that the IOL 40 is set on the setting stages 14a and 14b while the setting part 12 is opened (the half-split elements 12a and 12b are separated) as shown in Fig. 4A, the cartridge 10 is then mounted in the cylindrical unit 20 by closing the half-split elements 12a and 12b as shown in Fig. 4B, thereby applying stress on the set IOL 40 to bend or fold it to some extent.

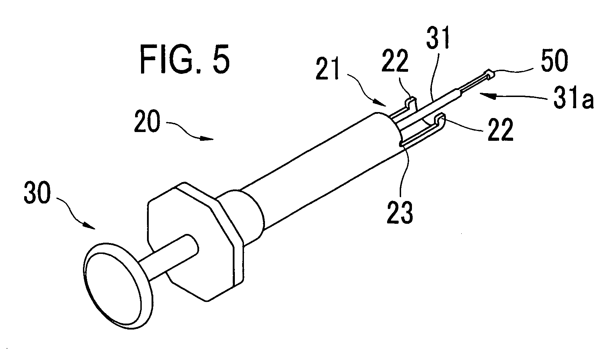

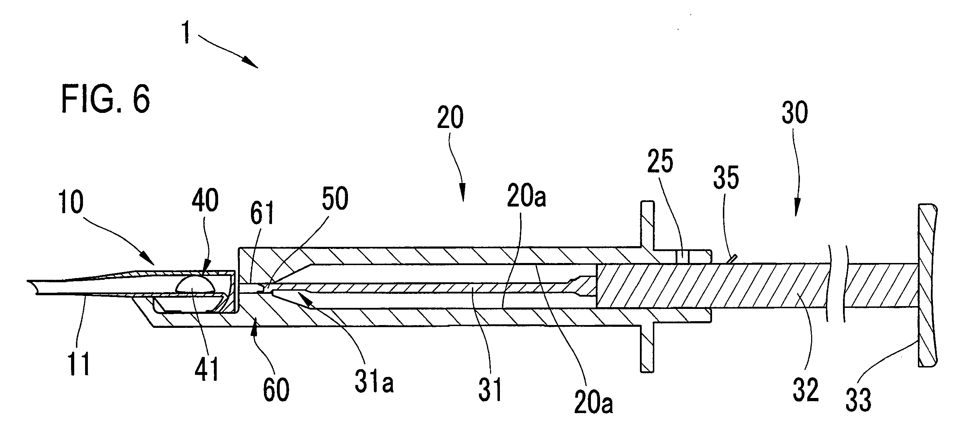

FIG. 5 is a specific perspective view showing the external structure of the cylindrical unit 20. The details of an internal structure of this cylindrical unit 20 are explained referring to Fig. 6. As shown in the figures, the cylindrical unit 20 is provided, at a front end, with a mounting part 21 in which the cartridge 10 is to be removably mounted. The mounting part 21 is of a nearly half split shape of the front end portion of the cylindrical unit 20. The mounting part 21 is formed with protrusions 22 at a front end and recesses 23 at a rear end, each of which are symmetric with respect to the push rod 31 extending along the central axis of the cylindrical unit 20. The protrusions 22 are positioned slightly above the central axis of the cylindrical unit 20 and have a distance therebetween slightly narrower (shorter) than the inner diameter of the cylindrical unit 20. Each protrusion 22 has a shape for a snap-in configuration serving as a guide for causing the opened half-split elements 12a and 12b to move in a closing direction when the cartridge 10 is mounted in the mounting part 21 and limiting the width of the half-split elements 12a and 12b, and also for reliably holding the mounted cartridge 10 against coming-off from the cylindrical 20. Right and left edges of the mounting part 21 also serve as a guide as with the protrusions 22 to cause the half-split elements 12a and 12b to close.

The push rod 31 is formed at its front end with a head portion 50 which will contact with (or hold) the optic part 41 of the IOL 40. A narrow portion 31a having a smaller diameter than the push rod 31 is formed between the head portion 50 and the push rod 31.

Next, the internal structure of the IOL injection instrument 1 assembled from the cartridge 10 and the cylindrical unit 20 is explained below. FIG. 6 is a schematic cross sectional view of the IOL injection instrument 1. In this figure, the cartridge 10 is mounted in the mounting part 21 and thus the IOL 40 is placed in a folded state.

As shown in Fig. 6, the inside of the cylindrical unit 20 is hollow, in which the plunger 30 is inserted so as to be movable forward and backward in an axial direction of the cylindrical unit 20. The plunger 30 includes the push rod 31, an axial base part ("base part") 32, and a press part 33. As with the cartridge 10, the cylindrical unit 20 and the plunger 30 are made as a disposable type which is to be thrown away after one use. Accordingly, each of the cylindrical unit 20 and the plunger 30 is made of resin or the like by integral molding. The support parts 42 are not illustrated in Fig. 6.

The press part 33 which is to be pressed by a user during the push-out operation of the IOL 40 and the base part 32 integrally formed at one end of the press part 33 are so rigid as to be less deformed by pressing by the user but to enable appropriate centering by the pressing mentioned later. Thus, the base part 32 is preferably formed with as large a diameter as possible. In this embodiment, the base part 32 is produced to have the same diameter as an inner wall 20a of the cylindrical unit 20. The base part 32 is therefore unlikely to cause axis deflection even if it moves forward and backward inside the cylindrical unit 20. The surfaces of the base part 32 and the inner wall 20 are mirror-finished to reduce friction or the like resulting from forward/backward movement of the plunger 30. In the above way, the centering mechanism of the push rod 31 is configured. At that time, the base of the push rod 31 (the boundary between the push rod 31 and the base part 32) is centered (positioned in place).

The push rod 31 is formed of an axial rod having a smaller diameter than the base part 32 and attached to the front end of the base part 32. When the base part 32 is moved forward, the IOL 40 is pushed forward from the cartridge 10 set in the front end of the cylindrical unit 20. The push rod 31 (the plunger 30) serves to move the IOL 40 from the front end of the cylinder to the outside. Accordingly, the push rod 31 is formed with such a diameter as to be passable through the inner passage in the injection part 11 and the cartridge 10 and be smaller than the base part 32. A front end portion of the push rod 31 includes a narrow portion 31a having a smaller diameter and a predetermined axial length to prevent the rear support part 42 of the IOL 40 set in the cartridge 10 from tangling (becoming caught) between the push rod 31 and the passage and hence becoming damaged when the IOL 40 is to be pushed out. Furthermore, the head portion 50 that will hold the IOL 40 is formed at a front end (an extreme forward end) of the narrow portion 31a. The narrow portion 31a has a length needed to avoid interference with the rear support part 42 (i.e., tangling of the support part 42) during push-out of the IOL 40.

The head portion 50 has a front end with protrusion and recess to catch the peripheral edge of the optic part 41 from above and below to hold the optic part 41. The head portion 50 in this embodiment is of a wedge-shaped cross section to scoop up the optic part 41. The diameter of the head portion 50 is designed to be equal to or smaller than the diameter of the push rod 31. The push rod 31 is narrower than the base part 32 and the narrow portion 31a is thinner than the push rod 31. Accordingly, a clearance for receiving the support part 42 is created between the push rod 31 and the passage (the inside of the cartridge 10 and others) and therefore the support part 42 is unlikely to be damaged by the plunger 30 during the push-out operation of the IOL 40.

The push rod 31 has such a restoring force as to restore to an original state on the push-out axis against the friction force occurring between the folded IOL 40 and the inner cylinder 11a even if the aforementioned axis deflection of the push rod 31 (displacement of the IOL 40) occurs in the standby position (the inner cylinder 11a) of the IOL 40. The axis deflection of the push rod 31 is caused when the push rod 31 warps or bends in a position forward than the centering portion 60 in association with displacement of the IOL 40. For the restoring force, the push rod 31 is made of a material having a mechanical property with bending strength of 60 to 150 MPa and bending modulus of 2000 to 3500 MPa. To be concrete, the push rod 31 is made of synthetic resin such as polycarbonate, ABS resin, PVC (polyvinyl chloride), acryl, and PEEK. The diameter of the push rod 31 has to be smaller than the inner diameter of the tip end of the injection part 11 in the passage of the injection part 11 and be enough to produce the above restoring force. To be concrete, the diameter of the push rod 31 is preferably 0.5 mm to 2.5 mm, more preferably 0.8 mm to 1.8 mm. In this embodiment, the plunger 30 (the head portion 50, the push rod 31, the base part 32, and the press part 33) is integrally formed.

In the cylindrical unit 20, at a place as near to the front end of the cylindrical unit 20 as possible (i.e., in the vicinity of the recess 23 in this embodiment), a centering portion 60 having a predetermined thickness in the axial direction is fixedly formed. As illustrated in the figure, the centering portion 60 has a back end formed in a cone shape whose open diameter is gradually smaller toward a front end formed with an inner cylinder (a fitting part) 61 having a diameter to allow the push rod 31 is fitted therein. Such cone shape makes easily fitting of the push rod 31 in the inner cylinder 61 during the push-out operation. When fitted in the inner cylinder 61, the push rod 31 is positioned and centered in the cylindrical unit 20. This can prevent the axis deflection of the push rod 31. The centering portion 60 also serves to restrict forward movement of the base part 32 during the push-out operation of the IOL 40. This makes it possible to prevent the head portion 50 from excessively coming out of the injection part 11 during the push-out operation. Thus, the IOL 40 can be injected in an eye appropriately. The centering portion 60 is integrally formed with the cylindrical unit 20.

The inner cylinder 61 is of an almost semi-cylindrical shape. The push rod 31 to be fitted in the inner cylinder 61 is also of the same semi-cylindrical shape as the inner cylinder 61. Accordingly, the push rod 31 is prevented from rotating (twisting rotation about the plunger) during the push-out operation.

During a moving operation of the plunger 30, the push rod 31 is fitted in the inner cylinder 61 and hence centered. At that time, the push rod 31 is fixed in the centering portion 60. On the basis of this fixed portion of the rod 31, the front end (the head portion 50) of the push rod 31 is restored.

Furthermore, the base part 32 is formed at its surface with a protrusion. The cylindrical unit 21 is formed at its inner wall with a recess engageable with the protrusion. The protrusion and the recess are configured to include a perceivable member for causing a user to perceive that the IOL 40 has reached or is about to reach the standby position during the moving operation of the plunger 30. For instance, the protrusion and the recess are configured to engage with each other and serve to cause a user to perceive the standby position of the IOL 40. In this embodiment, the protrusion is a plate spring (the perceivable member) 35 that can swing from the surface of the base part 32 to the outside. The plate spring 35 has a predetermined restoring force and is in an outward open state under no stress. The plate spring 35 is configured such that the plate spring 35 fits within the ridge line (the outer surface) of the base part 32 when the plate spring 35 is pressed against the surface of the base part 32. It is to be noted that the plate spring 35 is integrally formed with the plunger 30. In this embodiment, the recess is a through hole 25 formed in the rear side surface of the cylindrical unit 20. The through hole 25 has an inner diameter enough to receive the plate spring 35 in an open state. The through hole 25 and the plate spring 35 are placed in such positions that they engage with each other when the IOL 40 pressed by the head portion 50 reaches the standby position (the position of the inner cylinder 11a) during the moving operation of the plunger 30.

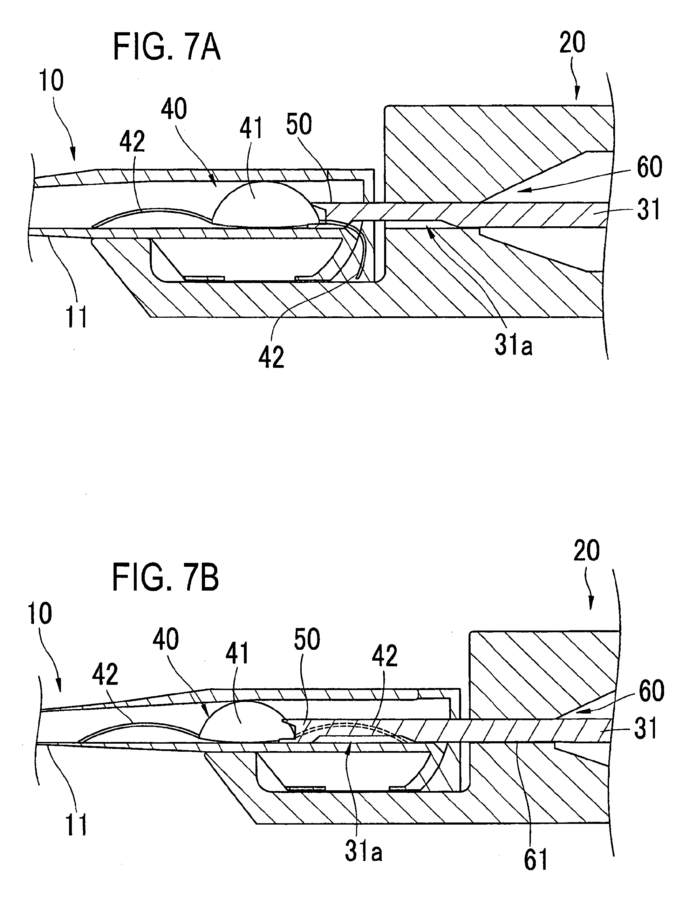

Next, the following explanation is given to the centering of the push rod 31 in association with the push-out operation of the IOL 40 (the moving operation of the push rod 31). FIGs. 7A and 7B are schematic cross sectional views of the vicinity of the centering portion 60. Specifically, FIG. 7A shows a state where the push rod 31 starts to fit in the centering portion 60 and FIG. 7B shows a state where the head portion 50 is in contact with the optic part 41. For facilitating explanation, the support parts 42 are illustrated on the cross sectional view and a portion thereof overlapping the head portion 50 and the narrow portion 31a is illustrated by a dotted line.

When the IOL 40 is folded in the cartridge 10, the front (anterior) support part 42 is spread forward along the inner wall of the cartridge 10. On the other hand, the rear (posterior) support part 42 hangs down, without receiving stress, from the rear end (the base end) of the cartridge 10. In this state, the rear support part 42 is preferably out of contact with the centering portion 60.

The distance from the rear end of the IOL 40 (the optic part 41) placed in the cartridge 10 to the front end of the centering portion 60 is determined to ensure a predetermined clearance for receiving the rear support part 42 when the IOL 40 is folded.

The length of the inner cylinder 61 is determined to adjust the fitting time of the push rod 31 with the inner cylinder 61 during the push-out operation. This distance is so long that, just before or when the head portion 50 reaches into contact with the optic part 41 during the push-out operation, the narrow portion 31a passes through the inner cylinder 61 and the push rod 31 fittable in the inner cylinder 61 enters the inner cylinder 61 from the entrance (the rear end) thereof. At that time, the entire passage of the inner cylinder 60 does not need to fit to the push rod 31 and a part of the push rod 31 has only to fit in at least the vicinity of the entrance of the inner cylinder 61.

The distance (length) from the front end of the head portion 50 to the rear end of the narrow portion 31a is determined to be so long enough to create a clearance around the narrow portion 31a (i.e. a clearance under the narrow portion 31a) for receiving the spread support part 42 to prevent the support part 42 from contacting (interfering) with the moving push rod 31 during the push-out operation.

With the above configuration, a centering sequence is carried out during the push-out operation of the IOL 40. The push rod 31 is first centered by the centering mechanism as mentioned above. In this state, when the push rod 31 is moved forward, the push rod 31 fits into the inner cylinder 61. Accordingly, the push rod 31 is centered at two points by the centering mechanism (the base part 32 and the cylindrical unit 20) and the centering portion 60. Alternatively, the push rod 31 is centered by only the inner cylinder 61. At that time, the head portion 50 comes into contact with the optic part 41. When the push rod 31 is further moved forward, the push rod 31 remains centered and the optic part 41 is caught by the head portion 50 and pushed into the injection part 11. Then, the optic part 40 and the head portion 50 reach the inner cylinder 11a which is the standby position.

Next, an explanation is given to the configuration for causing a user to perceive the standby position of the IOL 40 (the head portion 50) during the moving operation of the plunger 30.

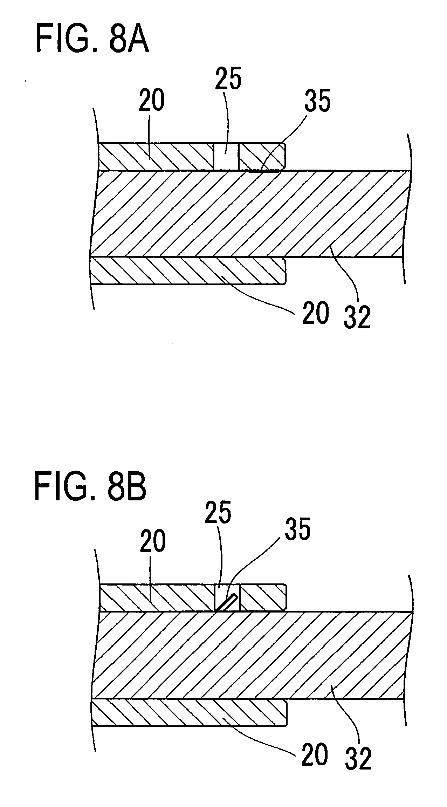

FIGs. 8 are enlarged cross sectional views of a rear part of the cylindrical unit 20. Specifically, FIG. 8A shows a state where the head portion 50 does not yet reach the standby position and FIG. 8B shows a state where the head portion 50 has advanced and reached the standby position. During the moving operation of the plunger 30, the plate spring 35 is pressed against the base part 32 by the cylindrical unit 20 (the state in FIG. 8A). This state is a closed state. It is to be noted that the plate spring 35 in the closed state completely fits within the ridge line of the base part 32 and thus causes little friction with the inner wall of the cylindrical unit 20. Therefore, the plate spring 35 have little influence on working pressure of the plunger 30. When the closed plate spring 35 reaches the through hole 25, it restores to the open state (the state in FIG. 8B). At that time, the behavior of the plate spring 35 is transmitted to the user who holds the plunger 30. Accordingly, the user perceives that the IOL 40 reaches the standby position. When the IOL 40 located in the standby position is pushed forward, the plate spring 35 is pressed by the cylindrical unit 20 but has little influence on the working pressure of the plunger 30.

The centering (restoring) of the head portion 50 (the push rod 31) having reached the standby position is explained below.

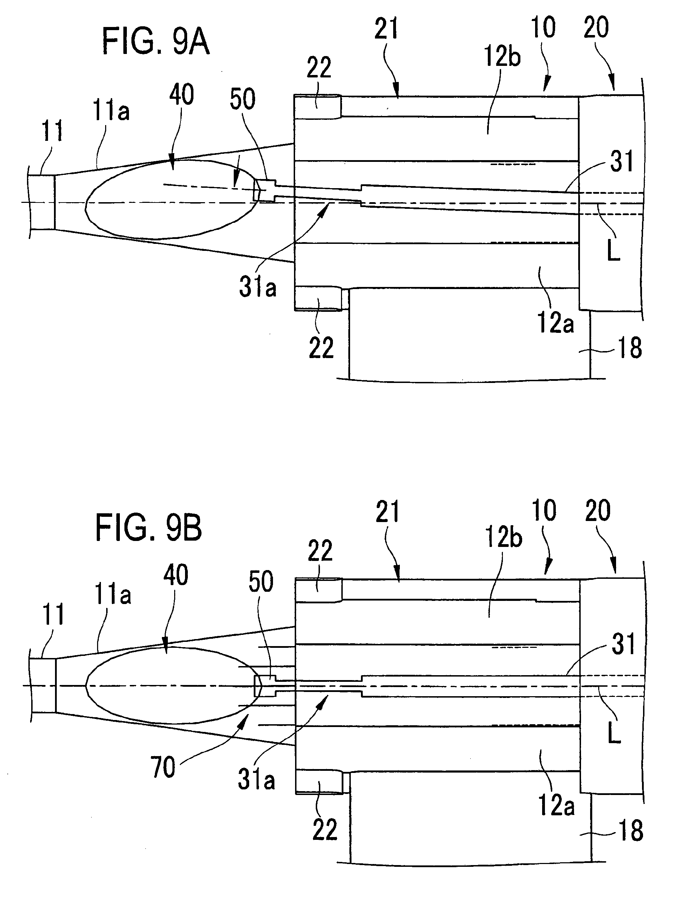

FIGs. 9 are enlarged top views of the cartridge 10 while the IOL 40 is located in the standby position. Specifically, FIG. 9A shows a state where the head portion 50 is off the push-out axis and FIG. 9B shows a state where the head portion 50 is on the push-out axis. For facilitating explanation, the covers 15a and 15b and the support parts 42 are not illustrated. In FIG. 9A, the mark 70 is not illustrated.

The push rod 31 and the head portion 50 centered by the centering portion 60 reach the standby position while they remain centered. However, there may be a case where the head portion 50 and the push rod 31 are deviated from the push-out axis L as shown in FIG. 9A. For instance, when the head portion 50 contacts with the IOL 40 and is further pushed forward, the position of the IOL 40 is displaced due to friction balance between the IOL 40 and the inner wall of the cartridge 10 (the inner cylinder 11a and the setting part 12) and other causes. At that time, the movement axis of the head portion 50 is also deflected. Herein, the IOL 40 slightly rotates in a lateral direction (a yaw direction) and the movement axis of the head portion 50 bends in the lateral direction. In other words, the push rod 31 bends in a position forward of the centering portion 60.

The IOL 40 located in the standby position is held with a clearance for allowing the lens 40 to slightly rotate inside the inner cylinder 11a. At that time, a portion of the push rod 31 fitted in the centering portion 60 is located on the push-out axis L. This position is referred to as a centering end. In the push rod 31, a force (an arrow in the figure) that returns the movement axis of the head portion 50 to the push-out axis L occurs from the centering end defined as a starting point. The restoring force of the push rod 31 exceeds the friction force occurring by contact between the IOL 40 located in the standby position and the inner wall of the inner cylinder 11a and causes the head portion 50 and the IOL 40 held in the head portion 50 to move toward the push-out axis L. At that time, the IOL 40 held in the head portion 50 is moved (rotated) inside the inner cylinder 11a, thereby correcting displacement of the IOL 40 and a bent state of the push rod 31 into respective appropriate states for push-out. Such centering of the head portion 50 by the push rod 31 is carried out in a few seconds from the time when the head portion 50 enters the standby position. During this period, the user checks the centering state of the head portion 50 by reference to the mark 70 serving as a reference for centering. Thus, the centering state of the head portion 50 can be correctly checked. Since the shape of the head portion 50 is designed to hold the IOL 40, the IOL 40 also can be moved and centered in association with the centering of the head portion 50.

In this way, the head portion 50 (the push rod 31) and the IOL 40 both being centered are pushed forward from the standby position, thereby pushing the IOL 40 out of the injection portion 11 while the IOL 40 remains centered.

As above, it is possible to easily push out the IOL by a simple configuration without increasing the number of components and prevent axis deflection. Furthermore, the IOL can be pushed out appropriately without using such a member that moves in sync with the push-out rod for centering and then separates therefrom later.

In the above embodiment, the inner cylinder 61 is formed in a uniform cross section to fit with the push rod 31 but is not limited thereto. It may have any configuration for centering the push rod 31 from the front end side of the centering portion 60. It may be a member that contacts with the support part 42 to the extent that no stress is applied on the support part 42 or a member that is located near to the extend that the member does not contact with the support parts 42. Accordingly, a thin fitting member (the inner cylinder) may be provided in each of the front end side and the rear end side of the centering portion 60.
In the above embodiment, the inner cylinder is provided in the centering portion but it is not limited thereto. The fitting part in which the push-out rod is to be fitted has only to be formed in the centering portion. For instance, a pair of pillar members arranged at such an interval that allows the push-out rod is engageable therebetween may be placed to face each other while the push-out rod is interposed therebetween. The pillar members may be formed to extend from top to bottom of the cylindrical unit or from bottom to top of the cylindrical unit. The pillar members may be formed in the cartridge. In such a case, the centering portion is formed in the cartridge. The pillar members may be disposed in plural pairs. The pillar members may be formed integral with the side wall.

Operations of the IOL injection instrument 1 having the above configuration are explained below. A user holds the cartridge 10 by grasping the grip 18 of the cartridge 10 by one hand and picking up the IOL 40 with forceps by the other hand. The user inserts the picked IOL 40 into the cartridge 10 from its base end and places it on the setting stages 14a and 14b. While no stress is applied on the cartridge 10, the half-split elements 12a and 12b are in an open state as shown in FIG. 4A. Accordingly, the IOL 40 set on the setting stages 14a and 14b is also retained in an unfolded state (a state where no stress is applied thereon) in the cartridge 10.

To mount the cartridge 10 in the cylindrical unit 20, the push rod 31 is firstly pulled toward the rear end of the cylindrical unit 20. The user engages the grip 18 (the rear end) of the cartridge 10 in the recess 23 of the mounting part 21 and pushes the bottom of the setting part 12 (the half-split elements 12a and 12b) against the protrusions 22 (or the right and left edges of the mounting part 21). When the bottom (the lower part) of the setting part 12 is pressed against the protrusions 22 (or the right and left edges of the mounting part 21), the protrusions 22 will guide the half-split elements 12a and 12b to close into contact with each other. As the setting part 12 is further pushed into the mounting part 21, the half-split elements 12a and 12b become mounted in a closed state in the mounting part 21 as shown in FIG. 4B.

In such a state where the half-split elements 12a and 12b are closed in contact with each other, the width (interval) of the setting stages 14a and 14b is narrow so that the wall surfaces of the setting stages 14a and 14b push the IOL 40 from right and left. The IOL 40 receives stress and thus is folded along the wall surfaces (setting surfaces) of the setting stages 14a and 14b.

The user then pours a viscoelastic material into the cartridge 10 to improve slidability of the IOL 40 in the cartridge 10. After the cartridge 10 is mounted in the mounting part 21, the user inserts the injection part 11 in the patient's eye from which a lens has been removed in advance and then pushes the press part 33 to move the base part 32 and the push rod 31 forward. The head portion 50 and the narrow portion 31a pass through the centering portion 60 and thus the push rod 31 is fitted in the inner cylinder 61 of the centering portion 60. Accordingly, the push rod 31 is centered and the head portion 50 integral with the push rod 31 pushes the IOL 40 forward from the set position into the injection part 11 while holding the side (edge) of the optic part 41 of the IOL 40. At that time, the plate spring 35 is pressed by the cylindrical unit 20.

When the plunger 30 is further pressed, the plate spring 35 comes to an open state in the through hole 25. This causes the user to perceive the behavior of the plate spring 35, so that the user can recognize that the head portion 50 is currently located in the standby position. The user checks the states of the IOL 40 and the head portion 50 both located in the standby position. In this state, the user checks the position of the head portion 50 with reference to the mark 70. At that time, there may be a case where the front end portion of the push rod 31 located forward of the centering portion 60 is warped or bent by unexpected displacement of IOL 40. If the push rod 31 is bent or warped as above and the movement axis of the head portion 50 is deflected, the push rod 31 is allowed to stand in this state. In this standby position, the IOL 40 is not completely folded and the restoring force of the push rod 31 obtained by its forming material and diameter exceeds the friction force caused by contact between the IOL 40 and the passage inner wall. Accordingly, some axis deflection of the push rod 31 can be corrected by its own restoring force. Consequently, the push rod 31 can return its axis onto the movement axis by its own restoring force while correcting the displacement of the IOL 40 into a normal state.

When the plunger 30 having been centered in the standby position is pushed, the IOL 40 is moved to the tip end side of the injection part 11. As the open diameter of the injection part 11 is smaller, the IOL 40 becomes folded (rolled) along the inner wall surface of the injection part 11. At this time, the support part 42 is spread. When the press part 33 is further pushed forward, the IOL 40 is pushed out of the tip end of the injection part 11.

In the embodiment mentioned above, the centering portion includes the inner cylinder having the same cross sectional shape as the push-out rod to push out the IOL with improved centering accuracy in the push-out operation of the IOL. However, the present invention is not limited to such configuration. The push-out rod and the centering portion may be formed in any shapes if only the push-out rod is not axially rotated and is appropriately centered. For instance, the push-out rod and the centering portion may be formed with a slit and a groove respectively, which are slidably engageable with each other.

In the above embodiment, the IOL 40 folded in the cartridge 10 is centered by the centering portion 60 and others and then is pushed out of the injection part 11 by the plunger 30, but the present invention is not limited to such configuration. As an alternative, while the IOL 40 under no stress is pushed by the centered plunger 30, the IOL 40 is folded by the inner configuration of the cartridge 10 and pushed out of the injection part 11.

In the above embodiment, the plate spring and the through hole cause the user to perceive the standby position of the head portion, but the present invention is not limited to such configuration. As an alternative, a protrusion and a recess engageable with each other may be adopted. Those protrusion and recess may be placed reversely. The protrusion is not limited to the plate spring and may be any component having a restoring force. The protrusion also may be formed in such a projecting shape as to have a little influence on the working pressure of the push-out member.

In the above embodiment, the head portion is of a protrusion and recess shape but is not limited thereto. The head portion may be configured in any shape that can contact with the IOL and push the IOL by movement of the push-out rod. The surface of the head portion which will contact with the IOL may be flat. In this case, the head portion comes into contact with the IOL with a certain level of friction force. Accordingly, even if the IOL is displaced in the standby position and the push-out rod is bent or warped, the displacement of the IOL is corrected by the restoration of the push-out rod.

In the above embodiment, the push-out member is pressed by the user to move backward/forward the push-out rod. However, the present invention is not limited to such configuration and may adopt any configuration that can push out the IOL by use of the injection part. As another alternative, the push-out member may be configured to be rotated about the axis by the user to move the push-out rod forward.
In the above embodiment, the IOL is placed in the cartridge when the IOL is to be injected. The present invention is not limited thereto and may be configured such that, for example, the IOL is placed and stored under no stress thereon in the cartridge for a long term. The present invention may also be applied to a pre-load type IOL injection instrument.

### Reference signs list

- 1: IOL injection instrument
- 10: Cartridge
- 11a: Inner cylinder
- 20: Cylindrical part
- 30: Push-out member (Plunger)
- 31: Push-out rod
- 40: Intraocular lens (IOL)
- 41: Optic part
- 50: Head portion
- 60: Centering part

## Claims

1. An IOL injection instrument (1) comprising:
a lens holding part (10) including: a setting part (12) having a lens set position on which an intraocular lens (40) is placed and a lens standby position provided forward of the lens set position; and a cylindrical injection part (11) coupled to the setting part (12) to feed the lens (40) placed on the setting part (12) from a tip end;
a main unit (20) having a cylindrical configuration provided with the lens holding part (10) at a front end; and
a plunger (30) provided to be movable forward and backward in an axis direction in a cylinder of the main unit (20) to push the lens out of the lens holding part (10), the plunger (30) including a push rod (31) formed at a front end with a head portion (50) which will contact with the optic part (41) of the lens (40),
the main unit (20) includes a centering portion (60) inter-nally fixedly formed therein to prevent axis deflection of the plunger (30), the centering portion (60) having a fitting part (61) having almost the same diameter as that of the plunger (30), and the centering portion (60) being fittable to the plunger (30) during a moving operation of the plunger (30) to center the plunger (30),
**characterized in that**
the push rod (31) is formed of a material having bending strength of 60 to 150 MPa and a bending modulus of 2000 to 3500 MPa and with the diameter of 0.5 mm to 2.5 mm as to produce a restoring force capable of correcting displacement and a bent state of the lens (40) against the friction force caused by contact between the lens (40) placed in the lens standby position and the inner wall of the lens holding part (10) in order to prevent the plunger (30) from bending in a position forward of the centering portion and deflecting an axis by displacement of the lens (40) when the lens (40) is moved from the lens set position to the lens standby position during the moving operation.

2. The IOL injection instrument (1) according to claim 1, wherein the plunger (30) is formed of polycarbonate or ABS resin.

3. The IOL injection instrument (1) according to claim 1 or 2, wherein a front end (50) of the plunger (30) has a protrusion and recess shape for holding the edge of the lens (40) from above and below.

4. The IOL injection instrument (1) according to any one of claims 1 to 3, wherein the lens holding part (10) includes a mark (70) allowing visual check of a centering position of the front end of the plunger (30) when it contacts with the lens (40) placed in the lens standby position.

5. The IOL injection instrument (1) according to claim 4, wherein the lens holding part (10) is made of an optically transparent material to allow external vision check of the lens (40) and the push rod (31) when they are placed in the lens holding part (10).

6. The IOL injection instrument (1) according to any one of claims 1 to 5, wherein the main unit (20) includes a mounting part (21) for removably mounting the lens holding part (10).

## Patentansprüche

1. IOL-Injektionsinstrument (1) mit:
einem Linsenhalteteil (10), der Folgendes aufweist:
einen Setzteil (12), der eine Linsensetzposition, an der eine Intraokularlinse (40) platziert wird, und eine Linsenruheposition hat, die vor der Linsensetzposition vorgesehen ist; und einen zylindrischen Injektionsteil (11),
der an dem Setzteil (12) gekoppelt ist, um die an dem Setzteil (12) platzierte Linse (40) von einem Spitzenende vorzuschieben;
einer Haupteinheit (20), die eine zylindrische Konfiguration hat, die an einem vorderen Ende mit dem Linsenhalteteil (10) versehen ist; und
einem Kolben (30), der so vorgesehen ist, dass er in einer axialen Richtung in einem Zylinder der Haupteinheit (20) nach vorn und nach hinten bewegbar ist, um die Linse aus dem Linsenhalteteil (10) herauszudrücken, wobei der Kolben (30) einen Druckstab (31) aufweist, der an einem vorderen Ende mit einem Kopfabschnitt (50) ausgebildet ist, der mit dem optischen Teil (41) der Linse (40) in Kontakt gelangt,
wobei die Haupteinheit (20) einen Zentrierabschnitt (60) aufweist, der darin intern befestigt ausgebildet ist, um eine Achsablenkung des Kolbens (30) zu verhindern, wobei der Zentrierabschnitt (60) einen Anbringungsteil (61) hat, der nahezu den gleichen Durchmesser wie der Kolben (30) hat, und wobei der Zentrierabschnitt (60) während eines Bewegungsbetriebs des Kolbens (30) an dem Kolben (30) anbringbar ist, um den Kolben (30) zu zentrieren,
**dadurch gekennzeichnet, dass**
der Druckstab (31) aus einem Material ausgebildet ist, das eine Biegefestigkeit von 60 bis 150 MPa und ein Biegemodul von 2000 bis 3500 MPa hat, und mit einem Durchmesser von 0,5 mm bis 2,5 mm, um so eine Reaktionskraft zu erzeugen, die eine Versetzung und einen Biegezustand der Linse (40) gegen die Reibkraft korrigieren kann, die durch einen Kontakt zwischen der Linse (40), die an der Linsenruheposition platziert ist, und der Innenwand des Linsenhalteteils (10) verursacht wird, um das Biegen des Kolbens (30) an einer Position vor dem Zentrierabschnitt und eine Abweichung einer Achse durch eine Versetzung der Linse (40) zu verhindern, wenn die Linse (40) von der Linsensetzposition zu der Linsenruheposition während des Bewegungsbetriebs bewegt wird.

2. IOL-Injektionsinstrument (1) gemäß Anspruch 1, wobei der Kolben (30) aus Polycarbonat oder einem ABS-Kunststoff ausgebildet ist.

3. IOL-Injektionsinstrument (1) gemäß Anspruch 1 oder 2, wobei ein vorderes Ende (50) des Kolbens (30) einen Vorsprung und eine ausgesparte Form hat, um die Kante der Linse (40) von oben und von unten zu halten.

4. IOL-Injektionsinstrument (1) gemäß einem der Ansprüche 1 bis 3, wobei der Linsenhalteteil (10) eine Markierung (70) aufweist, die eine visuelle Überprüfung einer Zentrierposition des vorderen Endes des Kolbens (30) ermöglicht, wenn dieser mit der Linse (40) in Kontakt ist, die an der Linsenruheposition platziert ist.

5. IOL-Injektionsinstrument (1) gemäß Anspruch 4, wobei der Linsenhalteteil (10) aus einem optisch transparenten Material besteht und eine externe visuelle Überprüfung der Linse (40) und des Druckstabs (31) ermöglicht, wenn diese in dem Linsenhalteteil (10) platziert sind.

6. IOL-Injektionsinstrument (1) gemäß einem der Ansprüche 1 bis 5, wobei die Haupteinheit (20) einen Montageteil (21) aufweist, um den Linsenhalteteil (10) abnehmbar zu montieren.

## Revendications

1. Instrument d'injection de lentille IOL (lentille intra-oculaire) (1) comprenant :
une partie de retenue de lentille (10) comportant : une partie de mise en place (12) présentant une position de mise en place de lentille, sur laquelle une lentille intra-oculaire (40) est placée et une position d'attente de lentille formée à l'avant de la position de mise en place de lentille ; et une partie d'injection cylindrique (11) couplée à la partie de mise en place (12) afin de délivrer la lentille (40) placée sur la partie de mise en place (12) à partir d'une extrémité en pointe ;
une unité principale (20) présentant une configuration cylindrique comportant la partie de retenue de lentille (10) au niveau d'une extrémité avant ; et
un piston (30) agencé de manière à pouvoir se déplacer vers l'avant et vers l'arrière suivant une direction axiale dans un cylindre de l'unité principale (20) afin de pousser la lentille hors de la partie de retenue de lentille (10), le piston (30) comportant une tige de poussée (31) comportant, au niveau d'une extrémité avant, une partie de tête (50) qui entre en contact avec la partie optique (41) de la lentille (40),
l'unité principale (20) comporte, formée à l'intérieur, une partie de centrage (60) fixée de manière interne de manière à empêcher une flexion axiale du piston (30), la partie de centrage (60) comportant une partie d'assemblage (61) présentant presque le même diamètre que celui du piston (30), et la partie de centrage (60) pouvant être assemblée sur le piston (30) au cours d'une opération de déplacement du piston (30) afin de centrer le piston (30),
**caractérisé en ce que** :
la tige de poussée (31) est formée en un matériau présentant une résistance à la flexion de 60 à 150 MPa et un module de flexion de 2000 à 3500 MPa et avec un diamètre de 0,5 mm à 2,5 mm de manière à produire un effort de rappel capable de corriger un déplacement et un état fléchi de la lentille (40) contre l'effort de frottement provoqué par le contact entre la lentille (40) placée dans la position d'attente de lentille et la paroi interne de la partie de retenue de lentille (10) afin d'empêcher le piston (30) de fléchir dans une position à l'avant de la partie de centrage et de s'écarter d'un axe par déplacement de la lentille (40) lorsque la lentille (40) est déplacée de la position de mise en place de lentille vers la position d'attente de lentille au cours de l'opération de déplacement.

2. Instrument d'injection de lentille IOL (1) selon la revendication 1, dans lequel le piston (30) est formé à base de polycarbonate ou de résine ABS.

3. Instrument d'injection de lentille IOL (1) selon la revendication 1 ou 2, dans lequel une extrémité avant (50) du piston (30) présente une forme en saillie et en creux de manière à retenir le bord de la lentille (40) par le dessus et le dessous.

4. Instrument d'injection de lentille IOL (1) : selon l'une quelconque des revendications 1 à 3, dans lequel la partie de retenue de lentille (10) comporte une marque (70) permettant un contrôle visuel d'une position de centrage de l'extrémité avant du piston (30) lorsqu'il est en contact avec la lentille (40) placée dans la position d'attente de lentille.

5. Instrument d'injection de lentille IOL (1) selon la revendication 4, dans lequel la partie de retenue de lentille (10) est réalisée en un matériau optiquement transparent afin de permettre un contrôle visuel externe de la lentille (40) et de la tige de poussée (31) lorsqu'elles sont placées dans la partie de retenue de lentille (10).

6. Instrument d'injection de lentille IOL (1) selon l'une quelconque des revendications 1 à 5, dans lequel l'unité principale (20) comporte une partie de montage (20) destinée à assurer le montage de manière amovible de la partie de retenue de lentille (10).
